# EUROPEAN PATENT SPECIFICATION

(11) **EP 2 238 115 B1**
(45) Date of publication and mention of the grant of the patent: **02.11.2011**
(21) Application number: 09708902.3
(22) Date of filing: 26.01.2009
(51) Int. Cl.: C07D 233/24, A61K 31/40, A61P 13/10

(54) **NOVEL IMIDAZOLINYLMETHYL ARYL SULFONAMIDES**
NEUARTIGE IMIDAZOLINYLMETHYL-ARYL-SULFONAMIDE
NOUVEAUX IMIDAZOLINYLMÉTHYLARYLSULFONAMIDES

(30) Priority: 04.02.2008 US 25836 P
(43) Date of publication of application: 13.10.2010
(73) Proprietor: F. Hoffmann-La Roche AG, 4070 Basel (CH)
(72) Inventor: O'YANG, Counde, Sunnyvale California 94087 (US); YASUDA, Dennis Mitsugu, Campbell California 95008 (US)
(74) Representative: Sauer, Frank
(86) International application number: PCT/EP2009/050837
(87) International publication number: WO 2009/098133

(56) References cited:
- US-B2- 6 756 395

## Description

This invention relates to an imidazolinylmethyl aryl sulfonamide which is an alpha-1A adrenergic partial agonist, associated pharmaceutical compositions, and methods for use as a therapeutic agent.

Alpha-1 adrenergic receptors (interchangeably named alpha-1 adrenoceptors) are G-protein coupled transmembrane receptors that mediate various actions of the sympathetic nervous system through the binding of the catecholamines, epinephrine and norepinephrine (NE). Currently, several subtypes of the alpha-1 adrenergic receptors are known to exist for which the genes have been cloned: alpha-1A (previously known as alpha-1C), alpha-1B and alpha-1D. The existence of a low affinity alpha-1 adrenoceptor for prazosin named alpha-1L, in human prostate has been determined. However, the gene for the alpha-1L adrenergic receptor subtype has yet to be cloned.

The alpha-1 adrenoceptor plays a part in the sympathetic maintenance of smooth muscle tone and alpha-1 adrenergic agonists are known to increase muscle tone in the lower urinary tract necessary for urine storage and urine emptying thus making adrenergic receptors important targets for drug development in urinary dysfunction (Testa, Eur.J.Pharmacol., 1993, 249, 307-315. Pharmacological studies resulting in the subdivision of alpha-1 adrenergic receptors have let to the suggestion that development of subtype-selective compounds may allow improved treatment with a lower incidence of side effects, and Tanaguchi et al., Eur.J.Pharmacol, 1996, 318, 117-122, have reported that compounds with selectivity for the alpha-1A receptor and to a lesser extent to the alpha-1L receptor over the alpha-1B and alpha-ID subtypes have selectivity for urethral over vascular tissue.

Urinary incontinence is a condition defined as the involuntary loss of urine to such an extent as to become a hygienic or social concern to the patient. Stress urinary incontinence (SUI) occurs when the internal sphincter does not close completely. The primary symptom is minor leakage from activities, such as coughing, sneezing, laughing, running, lifting, or even standing, that apply pressure to a full bladder. Leakage stops when the activity stops. SUI is most common in women between the ages of 25 and 50, and many regularly exercising women have some degree of SUI.

The methods presently available to treat SUI include physiotherapy and surgery. Treatment with pharmaceuticals is limited to the use of non-selective adrenergic agonists. Only a limited number of pharmaceutical agents have been employed, with varying success, to treat stress incontinence.

Phenylpropanolamine, pseudoephrine and midodrine are considered first-line therapy for mild to moderate stress incontinence (Wein, supra; Lundberg (editor), JAMA 1989, 261(18):2685-2690). These agents are believed to work both by direct activation of alpha-1 adrenoceptors and indirectly by displacement of endogenous norepinephrine from sympathetic neurons following uptake into the nerve terminal (Andersson and Sjogren, Progress in Neurobiology, 1982, 71-89). Activation of alpha-1 adrenoceptors located on the smooth muscle cells of the proximal urethra and bladder neck (Sourander, Gerontology 1990, 36:19-26; Wein, supra) evokes contraction and an increase in urethral closure pressure.

The utility ofphenylpropanolamine, pseudoephrine, and midodrine is limited by a lack of selectivity among the alpha-1 adrenoceptor subtypes and by the indirect action of these agents (i.e. activation of alpha-1, alpha-2, and beta-adrenoceptors in the central nervous system and periphery). As a result, any desired therapeutic effect of these agents may be accompanied by undesirable side effects such as an increase in blood pressure. The increase in blood pressure is dose-dependent and therefore limits the ability to achieve therapeutically effective circulating concentrations of these agents (Andersson and Sjogren, supra). Furthermore, in some patients these agents produce insomnia, anxiety and dizziness as a result of their central nervous system stimulant actions (Andersson and Sjogren, supra, Wein, supra).

Certain alpha-1A/1L agonists are known to be useful in treating various disease states including urinary incontinence, nasal congestion, sexual dysfunction such as ejaculation disorders and priapism, and CNS disorders such as depression, anxiety, dementia, senility, Alzheimer's, deficiencies in attentiveness and cognition, and eating disorders such as obesity, bulimia, and anorexia. See e.g. US Patent Nos. 5,952,362, 6,756,395, 6,852,726, and 6,979,696 which disclose a variety of 2-imidazolinylmethyl aryl and heteroaryl derivatives as alpha-1A/L agonists. Full agonists of the alpha 1A/1L adrenoceptor subtype, while potentially effective at treating urinary incontinence, can be limited by undesirable cardiovascular and central nervous system side effects. Selective alpha 1A/1L receptor modulators with reduced intrinsic efficacy (i.e., "partial agonists") can reduce such side effects while maintaining the contractile effects on urethral smooth muscle needed for treating incontinence.

Due to side effects and /or limited efficacy associated with the current available medicaments, there is an unmet medical need for useful compounds. A compound having the desired alpha-1A adrenergic partial agonist profile is desirable.

In one aspect, the application provides a compound of formula I: 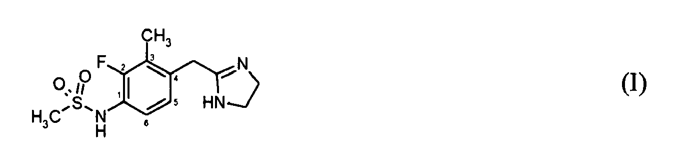 or a pharmaceutically acceptable salt or prodrug thereof.

The compound of Formula I, N-[4-(4,5-Dihydro-1H-imidazol-2-ylmethyl)-2-fluoro-3-methylphenyl]-methanesulfonamide (nomenclature used in this Application is based on AUTONOM™ v.4.0), has been found to exhibit unexpectedly enhanced selectivity, for enhancement of intraurethral pressure (IUP) over blood pressure (MAP), as a partial agonist of alpha-1A adrenoceptors. The combination of the fluoro and methyl substituents on the 2- and 3-position of the phenyl ring, respectively, provide unexpected advantages over the general class of imidazolinylmethyl aryl sulfonamides in that it has both a favorable intrinsic activity, or efficacy, as a partial agonists, which is ideally between 0.35 to 0.60, of 0.46 and an affinity, or pEC50 value, of 6.2. As full agonist activity is undesirable due to hypertension related side effects, the combination of high affinity and partial agonist behavior is critical for optimization of urethral activity benefits associated with effective modulation of alpha-1A adrenoceptors coupled with minimization of diastolic blood pressure related side effects. Furthermore, the compound of Formula I, in comparison to analogue compounds, exhibits improved durability of IUP response over time which is necessary for effective treatment of incontinence.

In one embodiment, the application provides the compound of formula I, wherein the pharmaceutically acceptable salt is hydrochloride.

In one embodiment, the application provides a composition comprising the compound of formula 1 and further comprising a pharmaceutically acceptable carrier.

In one embodiment, the application provides the above composition, wherein the composition is suitable for administration to a subject having a disease state which is alleviated by treatment with an alpha-1A receptor partial agonist.

In one embodiment, the application provides a method for preventing, alleviating, or treating a disorder modulated by alpha-1A adrenoceptors, said method comprising administering to a subject in need thereof an effective amount of the compound of formula 1.

In one embodiment, the application provides the above method, wherein the disorder is selected from urge incontinence, stress incontinence, overflow incontinence, and functional incontinence.

In one embodiment, the application provides a method for preventing, alleviating, or treating a disorder modulated by alpha-1A adrenoceptors, wherein the disorder is stress incontinence.

In one embodiment, the application provides a method for preventing, alleviating, or treating a disorder modulated by alpha-1A adrenoceptors, wherein the disorder is urge incontinence.

In one embodiment, the application provides a method for preventing, alleviating, or treating a disorder modulated by alpha-1A adrenoceptors, wherein the disorder is overflow incontinence.

In one embodiment, the application provides a method for preventing, alleviating, or treating a disorder modulated by alpha-1A adrenoceptors, wherein the disorder is functional incontinence.

In one embodiment, the application provides a method for preventing, alleviating, or treating a disorder modulated by alpha-1A adrenoceptors, said method comprising administering to a subject in need thereof an effective amount of the compound of formula 1 in combination with a second modulator of alpha-1A adrenoceptors.

In one embodiment, the application provides a method of treating or preventing a disease state characterized by urinary incontinence comprising administering to a subject in need thereof an effective amount of a compound of formula 1.
Figure 1. Depiction of Data Measurement in Anesthetized Rabbit Model
Figure 2. Formula I in Anesthetized Rabbit Model
Figure 3. Analogue Compound in Anesthetized Rabbit Model
Figure 4. Analogue Compound in Anesthetized Rabbit Model
Figure 5. Analogue Compound in Anesthetized Rabbit Model
Figure 6. Analogue Compound in Anesthetized Rabbit Model
Figure 7. Analogue Compound in Anesthetized Rabbit Model

Unless otherwise stated, the following terms used in this Application, including the specification and claims, have the definitions given below. It must be noted that, as used in the specification and the appended claims, the singular forms "a", "an," and "the" include plural referents unless the context clearly dictates otherwise.

All patents and publications identified herein are incorporated herein by reference in their entirety.

As used herein, "IUP" means intraurethral pressure and is measured as the 2 minute mean from the first peak of the urethral response.

As used herein, "MAP" means mean arterial blood pressure and is measured as the average blood pressure during the 2 minute section where IUP is measured.

As used herein, "durability of IUP response over time" means the slope of the IUP response in mmHg/min and is calculated immediately after the 2 minute IUP response for 5 minutes (2-7 minutes post the first peak) for the top 3 doses.

"Aryl" means the monovalent cyclic aromatic hydrocarbon radical consisting of one or more fused rings in which at least one ring is aromatic in nature, which can optionally be substituted with hydroxy, cyano, lower alkyl, lower alkoxy, alkylthio, halo, haloalkyl, hydroxyalkyl, nitro, alkoxycarbonyl, amino, alkylamino, dialkylamino, aminocarbonyl, carbonylamino, aminosulfonyl, sulfonylamino, nitro, and/or alkylsulphonyl, unless otherwise indicated. Examples of aryl radicals include, but are not limited to, phenyl, naphthyl, biphenyl, indanyl, anthraquinolyl, and the like.

"Pharmaceutically acceptable" means that which is useful in preparing a pharmaceutical composition that is generally safe, non-toxic, and neither biologically nor otherwise undesirable and includes that which is acceptable for veterinary as well as human pharmaceutical use.

"Arylsulfonyl" means a radical -S(O)₂R where R is an aryl group as defined herein.

"2-Imidazolinylmethyl", "imidazolin-2-ylmethyl", "imidazolinylmethyl", and 4,5-dihydro-1H-imidazol-2-ylmethyl", which may be used interchangeably, mean the moiety designated by the structure

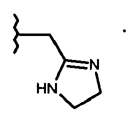

It is to be understood that the double bond in 2-imidazoline and 2-imidazolinylmethyl may assume other resonance forms. The terms 2-imidazoline 2-imidazolinylmethyl include all such resonance forms.

"Isomerism" means compounds that have identical molecular formulae but that differ in the nature or the sequence of bonding of their atoms or in the arrangement of their atoms in space. Isomers that differ in the arrangement of their atoms in space are termed "stereoisomers". Stereoisomers that are not mirror images of one another are termed "diastereoisomers", and stereoisomers that are non-superimposable mirror images are termed "enantiomers", or sometimes optical isomers. A carbon atom bonded to four nonidentical substituents is termed a "chiral center".

"Chiral compound" means a compound with one or more chiral center. It has two enantiomeric forms of opposite chirality and may exist either as an individual enantiomer or as a mixture of enantiomers. A mixture containing equal amounts of individual enantiomeric forms of opposite chirality is termed a "racemic mixture". A compound that has more than one chiral center has 2ⁿ⁻¹ enantiomeric pairs, where n is the number of chiral centers. Compounds with more than one chiral center may exist as either an individual diastereomer or as a mixture of diastereomers, termed a "diastereomeric mixture". When chiral centers are present, the stereoisomers may be characterized by the absolute configuration (R or S) of the chiral centers. Absolute configuretion refers to the arrangement in space of the substituents attached to a chiral center. The substituents attached to a chiral center under consideration are ranked in accordance with the *Sequence Rule* of Cahn, Ingold and Prelog. (Cahn et al. Angew. Chem. Inter., 1966, Edit., 5, 385; errata 511; Cahn et al. Angew. Chem., 1966, 78, 413; Cahn and Ingold, J. Chem. Soc. (London), 1951, 612; Cahn et al., Experientia, 1956, 12, 81; Cahn, J., Chem.Educ., 1964, 41, 116).

"Tautomers" refers to compounds whose structures differ markedly in arrangement of atoms, but which exist in easy and rapid equilibrium. It should also be understood that when compounds have tautomeric forms, all tautomeric forms are intended to be within the scope of the invention, and the naming of the compounds does not exclude any tautomer form.

"Pharmaceutically acceptable salts" of a compound means salts that are pharmaceutically acceptable, as defined herein, and that possess the desired pharmacological activity of the parent compound. Such salts include:
(1) acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid, and the like; or
(2) salts formed when an acidic proton present in the parent compound either is replaced by a metal ion, e.g., an alkali metal ion, an alkaline earth ion, or an aluminum ion; or coordinates with an organic or inorganic base. Acceptable organic bases include diethanolamine, ethanolamine, N-methylglucamine, triethanolamine, tromethamine, and the like. Acceptable inorganic bases include aluminum hydroxide, calcium hydroxide, potassium hydroxide, sodium carbonate and sodium hydroxide.

It should be understood that all references to pharmaceutically acceptable salts include solvent addition forms (solvates) or crystal forms (polymorphs) as defmed herein, of the same acid addition salt.

The preferred pharmaceutically acceptable salts are the salts formed from acetic acid, hydrochloric acid, sulphuric acid, methanesulfonic acid, maleic acid, phosphoric acid, tartaric acid, citric acid, sodium, potassium, calcium, zinc, and magnesium.

"Solvates" means solvent additions forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

"Subject" means mammals and non-mammals. Mammals means any member of the Mammalia class including, but not limited to, humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" does not denote a particular age or sex.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgement of the attending medical or veterinary practitioner, and other factors.

"Pharmacological effect" as used herein encompasses effects produced in the subject that achieve the intended purpose of a therapy. For example, a pharmacological effect would be one that results in the prevention, alleviation or reduction of urinary incontinence in a treated subject.

"Disease state" means any disease, condition, symptom, or indication.

"Treating" or "treatment" of a disease state includes:
(1) preventing the disease state, i.e. causing the clinical symptoms of the disease state not to develop in a subject that may be exposed to or predisposed to the disease state, but does not yet experience or display symptoms of the disease state;
(2) inhibiting the disease state, i.e., arresting the development of the disease state or its clinical symptoms; or
(3) relieving the disease state , i.e., causing temporary or permanent regression of the disease state or its clinical symptoms.

"α₁-adrenergic receptors", "α_{1A}-adrenergic receptors" (previously known as "α_{1C}-adrenergic receptors"), "α_{1L}-adrenergic receptors", or "α_{1A}/_{1L}-adrenergic receptors", which may be used interchangeably with "α₁-adrenoceptors", "α_{1A}-adrenoceptors" (previously known as "α_{1C}-adrenoceptors receptors"), "α_{1L}-adrenoceptors" or "α_{1A}/_{1L}-adrenoceptors" respectively, refers to a molecule conforming to the seven membrane-spanning G-protein receptors, which under physiologic conditions mediate various actions, e.g., in the central and/or peripheral sympathetic nervous system through the binding of the catecholamines, epinephrine and norepinephrine.

"Agonist" or "full agonist" means a molecule, such as a compound, a drug, an enzyme activator, or a hormone, that enhances the activity of another molecule or receptor site.

"Partial agonist" means activates a receptor, but only produces a partial physiological response compared to a full agonist.

"Urinary Incontinence" is a condition characterized by the involuntary loss of urine, which is objectively demonstrable. It is both a social and hygienic problem. Stated simply, incontinence results from the failure of the bladder and/or the urethra to work properly, or when the coordination of their functions is defective. While the prevalence of incontinence is two-fold higher in females, with the greatest incidence in postmenopausal women, it also affects males.

Urinary incontinence can be classified into four basic types: urge, stress, overflow and functional, and as used herein the term "urinary incontinence" encompasses all four types.

Urge incontinence (detrusor instability) is the involuntary loss of urine associated with a strong urge to void. This type of incontinence is the result of either an overactive or hypersensitive detrusor muscle. The patient with detrusor overactivity experiences inappropriate detrusor contractions and increases in intravesical pressure during bladder filling. Detrusor instability resulting from a hypersensitive detrusor (detrusor hyperreflexia) is most often associated with a neurological disorder.

Genuine stress incontinence (outlet incompetence) is the involuntary loss of urine occurring when increases in intra-abdominal pressure cause a rise in intravesical pressure which exceeds the resistance offered by urethral closure mechanisms. Stress incontinent episodes can result from normal activities such as laughing, coughing, sneezing, exercise, or, in severe stress incontinent patients, standing or walking. Physiologically, stress incontinence is often character-rized by a descensus of the bladder neck and funneling of the bladder outlet. This type of incontinence is most common in multiparous women, as pregnancy and vaginal delivery can cause loss of the vesicourethral angle and damage to the external sphincter. Hormonal changes associated with menopause may exacerbate this condition.

Overflow incontinence is an involuntary loss of urine resulting from a weak detrusor or from the failure of the detrusor to transmit appropriate signals (sensory) when the bladder is full. Overflow incontinent episodes are characterized by frequent or continuous dribbling of urine and incomplete or unsuccessful voiding.

Functional incontinence, in contrast to the types of incontinence described above, is not defined by an underlying physiological dysfunction in the bladder or urethra. This type of incontinence includes the involuntary loss of urine resulting from such factors as decreased mobility, medications (e.g., diuretics, muscarinic agents, or alpha-1 adrenoceptor antagonists), or psychiatric problems such as depression or cognitive impairment.

"A method of treating or preventing incontinence" refers to the prevention of or relief from the symptoms of incontinence including involuntary voiding of feces or urine, and dribbling or leakage of feces or urine which may be due to one or more causes including, but not limited to, pathology altering sphincter control, loss of cognitive function, overdistention of the bladder, hyper- reflexia and/or involuntary urethral relaxation, weakness of the muscles associated with the bladder, or neurologic abnormalities.

In general, the nomenclature used in this Application is based on AUTONOM™ v.4.0, a Beilstein Institute computerized system for the generation of IUPAC systematic nomenclature. Chemical structures shown herein were prepared using ISIS^{®} version 2.4. Any open valency appearing on a carbon, oxygen, sulfur or nitrogen atom in the structures herein indicates the presence of a hydrogen atom. Whenever a chiral carbon is present in a chemical structure, it is intended that all stereoisomers associated with that chiral carbon are encompassed by the structure. Whenever a chemical structure shown herein can exist in a different tautomeric form, it is intended that the structure encompasses such different tautomeric forms.

The following preparations and examples are given to enable those skilled in the art to more clearly understand and to practice the present invention. They should not be considered as limiting the scope of the invention, but merely as being illustrative and representative thereof.

The compound of the present invention may be made by the methods depicted in the illustrative synthetic reaction schemes shown and described below.

The starting materials and reagents used in preparing Formula I generally are either available from commercial suppliers, such as Aldrich Chemical Co., or are prepared by methods known to those skilled in the art following procedures set forth in standard references. Where necessary, conventional protecting group techniques were used as described by Greene et al., Protecting Groups in Organic Synthesis, 3rd Ed., Wiley Interscience, 1999. The following synthetic reaction schemes are merely illustrative of some methods by which the compound of the present invention may be synthesized, and various modifications to these synthetic reaction schemes may be made and will be suggested to one skilled in the art having referred to the disclosure contained in this Application.

The starting materials and the intermediates of the synthetic reaction schemes may be isolated and purified if desired using conventional techniques, including but not limited to filtration, distillation, crystallization, chromatography, and the like. Such materials may be characterized using conventional means, including physical constants and spectral data.

Unless specified to the contrary, the reactions described herein preferably take place at atomspheric pressure over a temperature range from about -78°C to about 150°C, more preferably from about 0°C to about 125°C, and most preferably and conveniently at about room (or ambient) temperature (RT), e.g., about 20°C.

### Chemical Syntheses

### A. Preparation of 1,3-difluoro-2-methyl-4-nitro-benzene (1)

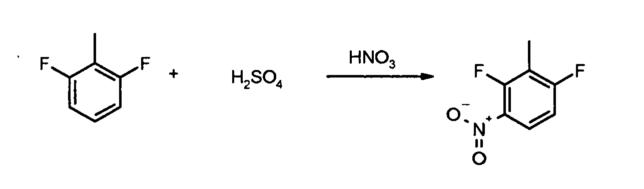

24.6mL (0.60mol) of fuming nitric acid was slowly added over 45 minutes to 61.5g (0.48mol) of 1,3-difluoro-2-methyl-benzene in 78mL (1.463mol) concentrated sulfuric acid in a partial ice bath at 15-20°C, and maintaining the reaction temperature, upon addition, between 25-30°C. The reaction mixture was then allowed to stir for an additional 1h at RT and subsequently poured into 1L ice water, filtered, washed with 3x300mL water, and dried *in vacuo* to yield 49.93g crude product as a white solid. The crude product was then crystallized from 60mL hexane, filtered, and solvent removed *in vacuo* to yield 41.260g (49%) product as a yellow crystalline solid and a further 7.58g product was isolated from the mother liquor.

### B. Preparation of Cyano-(3-fluoro-2-methyl-4-nitro-phenyl)-acetic acid tert-butyl ester (2)

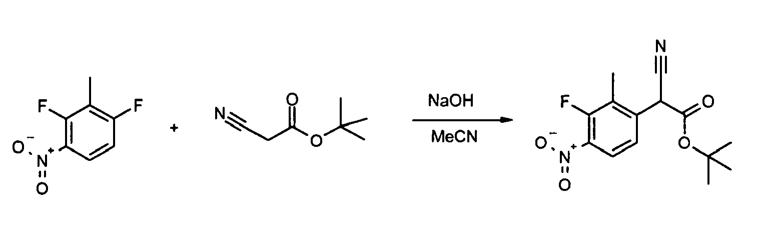

20.2g (0.505mol) NaOH was added to 35.05g (0.202mol) 1,3-difluoro-2-methyl-4-nitro-benzene and 31.39g (0.222mol) t-butyl cyanoacetate in 255mL MeCN under N₂ and cooled in an ice bath. The mixture was allowed to stir at 5°C for 1.5h prior to removing from ice bath. The mixture was subsequently poured into 350g ice water/100mL EtOAc, acidified with conc. HCl to pH 1, added to 300mL EtOAc, separated, washed with 500mL brine, dried over MgSO₄, filtered, and solvent *removed in vacuo* to yield 56.98g product as a brown oil.

### C. Preparation of (3-Fluoro-2-methyl-4-nitro-phenyl)-acetonitrile (3)

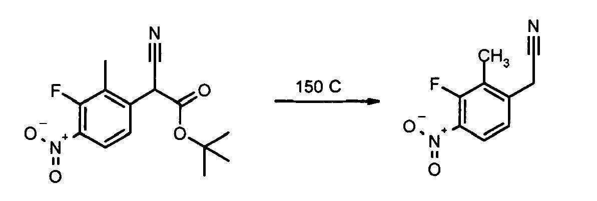

36.23g cyano-(3-fluoro-2-methyl-4-nitro-phenyl)-acetic acid tert-butyl ester was heated to 150°C *in vacuo* for 3h and allowed to cool to RT. The mixture was purified on a silica gel column, eluting a first time with 30-40% EtOAc/hexane and a second time with 16-35% EtOAc/hexane to give 7.66g product (19%).

### D. Preparation of (4-Amino-3-fluoro-2-methyl-phenyl)-acetonitrile (4)

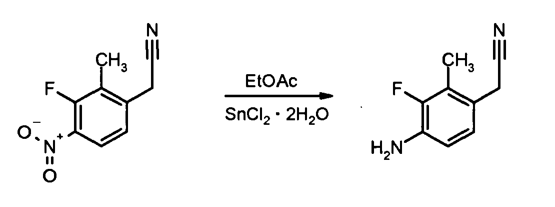

35.05g (0.55mol) SnCl₂·2H₂O was added to 7.54g (0.0388mol) (3-Fluoro-2-methyl-4-nitrophenyl)-acetonitrile in 130mL EtOAc and heated to 70°C under N₂ for 2h and cooled to RT and poured into 400mL EtOAc, to which 500mL saturated NaHCO₃ was slowly added. The layers were separated and the aqueous layer was extracted into 3x300mL EtOAc, dried over MgSO₄, filtered, and solvent *removed in vacuo* to yield 6.306g crude product which was purified on a silica gel column eluting with 26-35% EtOAc/hexane to yield 4.687g (73%) pure product.

### E. Preparation of N-(4-Cyanomethyl-2-tluoro-3-methyl-phenyl)-methanesulfonamide (5)

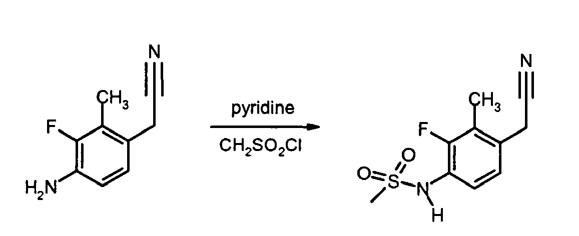

2.63mL (0.039mo1)of MsCl was added to 4.65g (0.0283mol) (4-Amino-3-fluoro-2-methylphenyl)-acetonitrile in 200mL pyridine under N₂ and cooled in an ice bath at 5°C for 1h and then for 3h at RT. The mixture was then poured into 50g ice, acidified with conc. HCl to pH 1, extracted with 200mL EtOAc once and again with 100mL EtOAc, washed with brine, dried over MgSO₄ filtered, and solvent *removed in vacuo* to yield crude product that was crystallized from 35mL EtOAc to yield 5.66g of 88% pure product.

### F. Preparation of N-[4-(4,5-Dihydro-1H-imidazol-2-ylmethyl)-2-fluoro-3-methyl-phenyl]methanesulfonamide (Formula I)

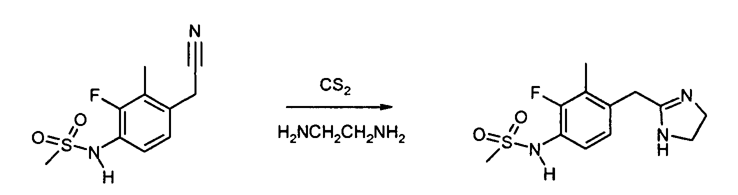

5.03g (0.0207mol) N-(4-cyanomethyl-2-fluoro-3-methyl-phenyl)-methanesulfonamide was dissolved in 15mL ethylene diamine in a microwave tube to which 3µL CS₂ were added, the tube sealed, heated in the microwave at 135°C for 11h. The solvent was then removed *in vacuo,* the residue taken up in MeOH, the solvent *removed in vacuo* at 50°C, 50mL MeOH added, to yield a precipitate which was dissolved in 220 mL hot MeOH, evaporated to 50mL and allowed to cool in refrigerator, filtered, and solvent *removed in vacuo* to yield 3.978g product (67%).

### G. Preparation of N-[4-(4,5-Dihydro-IH-imidazol-2-ylmethyl)-2-fluoro-3-methyl-phenyllmethanesulfonamide hydrochloride (hydrochloride salt of Formula I)

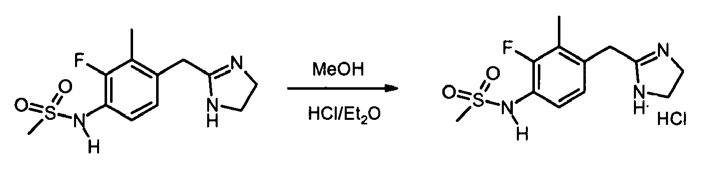

3.96g (0.0138) N-[4-(4,5-dihydro-1H-imidazol-2-ylmethyl)-2-fluoro-3-methyl-phenyl]-methane-sulfonamide suspended in 50mL MeOH was treated with 16.6mL of 1M HCUether to dissolve the solid material. The solvent was then removed *in vacuo* and 40mL MeOH was added to dissolve the residue, the solvent evaporated to give crude product which was crystallized from 10mL hot MeOH/10mL ether, refrigerated, filtered and subsequently dried extensively (3 weeks) *in vacuo* at 80-95°C to give 3.388g product (1.21% MeOH) (MP = 214-214.6° C, MS [M+H]⁺ = 286; CHN Calcd: C (44.79%), H (5.32%), N (13.06%); Found: C (44.53), H (5.42), N (12.79)).

The compound of the present invention have selective alpha-1A adrenergic selective activity and as such are expected to be useful in the treatment of various disease states, such as urinary incontinence; nasal congestion; sexual dysfunction, such as ejaculation disorders and priapism; CNS disorders such as depression, anxiety, dementia, senility, Alzheimer's, deficiencies in attentiveness and cognition, and eating disorders such as obesity, bulimia, and anorexia.

Urinary incontinence (UI) is a condition defined as the involuntary loss of urine to such an extent as to become a hygienic or social concern to the patient. Involuntary loss of urine occurs when pressure inside the bladder exceeds retentive pressure of the urethral sphincters (intraurethral pressure). Four major types of urinary incontinence have been defined based on symptoms, signs and condition: stress, urge, overflow and functional incontinence.

Stress urinary incontinence (SUI) is the involuntary loss of urine during coughing, sneezing, laughing, or other physical activities. The present methods to treat SUI include physiotherapy and surgery. Treatment with pharmaceutical agents is limited to the use of non selective-adrenergic agonists like phenylproanolamine and midodrine. The rationale for the use of adrenergic agonists for the treatment of SUI is based on physiological data indicating an abundant noradrenergic input to smooth muscle of the urethra.

Urge incontinence (detrusor instability) is the involuntary loss of urine associated with a strong urge to void. This type of incontinence is the result of either an overactive or hypersensitive detrusor muscle. The patient with detrusor overactivity experiences inappropriate detrusor contractions and increases in intravesical pressure during bladder filling. Detrusor instability resulting from a hypersensitive detrusor (detrusor hyperreflexia) is most often associated with a neurological disorder.

Overflow incontinence is an involuntary loss of urine resulting from a weak detrusor or from the failure of the detrusor to transmit appropriate signals (sensory) when the bladder is full. Overflow incontinent episodes are characterized by frequent or continuous dribbling of urine and incomplete or unsuccessful voiding.

Functional incontinence, in contrast to the types of incontinence described above, is not defined by an underlying physiological dysfunction in the bladder or urethra. This type of incontinence includes the involuntary loss of urine resulting from such factors as decreased mobility, medications (e.g., diuretics, muscarinic agents, or alpha-1 adrenoceptor antagonists), or psychiatric problems such as depression or cognitive impairment.

The compound of this invention are also particularly useful for the treatment of nasal congestion associated with allergies, colds, and other nasal disorders, as well as the sequelae of congestion of the mucous membranes (e.g., sinusitis and otitis media). with less or no undesired side effects.

These and other therapeutic uses are described, e.g., in Goodman & Gilman's, The Pharmacological Basis of Therapeutics, ninth edition, McGraw-Hill, New York, 1996, Chapter 26:601-616; and Coleman, R.A., Pharmacological Reviews, 1994, 46:205-229.1

### General Strategy for Testing Alpha-1A adrenoceptor Partial Agonists:

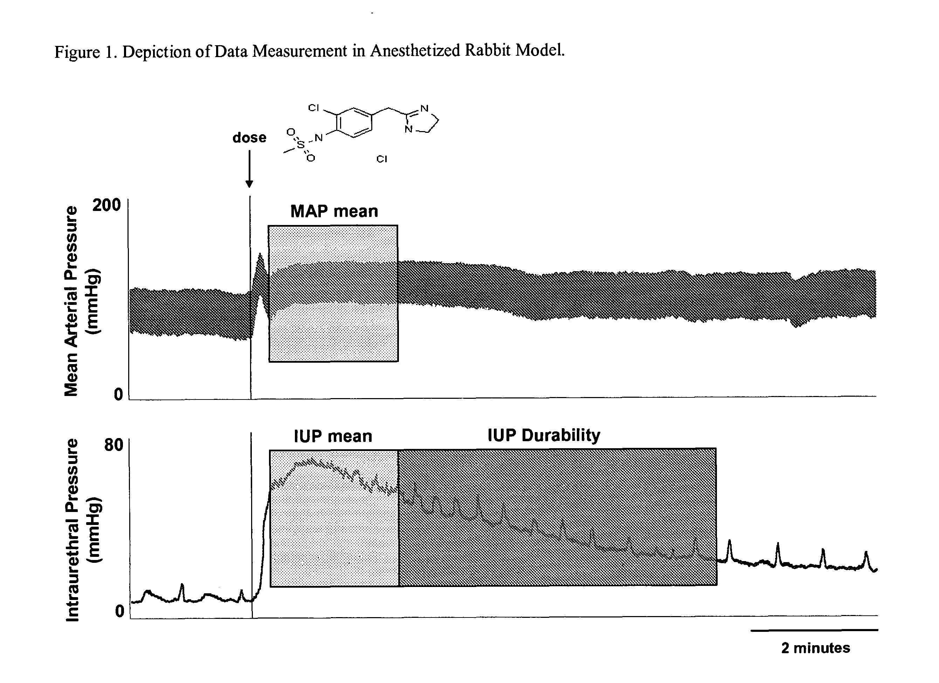

In general, IUP is the intraurethral pressure and is measured as the 2 minute mean from the first peak of the urethral response (Figure 1). MAP is the mean arterial blood pressure and is measured as the average blood pressure during the 2 minute section where IUP is measured. The durability is the slope of the IUP response in mm Hg/min and is calculated immediately after the 2 minute IUP response for 5 minutes (2-7 minutes post the first peak) for the top 3 doses.

### ANESTHETIZED RABBIT Model

**Surgery**: Female, Dutch Belted rabbits (1.20 - 2.0 kg, Myrtle's Rabbitry, TN) were anesthetized with isoflurane (3.0% at 2 to 4 L/min) and urethane (1.5 grams/kg, s.c.). In preparation for surgery, the rabbits were shaved, scrubbed (i.e., perineal area, ventral neck, and ventral, caudal surface of the abdomen) and were administered Ringers Lactate Solution (s.c.) to maintain fluids. The femoral vein and carotid artery were isolated and cannulated with PE-50 and PE-90 tubing (Becton- Dickinson), respectively, for the administration of drugs (vein) and the measurement of blood pressure (artery). An abdominal incision was made, exposing the ureters and the bladder. The ureters were isolated and cannulated proximal to the urinary bladder with PE-50 tubing, to drain urine from the kidneys. The urethra was isolated and catheterized via the bladder dome with an 8-French solid state single sensor transducer catheter (Unisensor USA Inc.) with the sensor located at the tip of the catheter. The sensor was placed at a level just beyond the pubic bone, distal to the bladder dome and secured to the bladder dome with silk suture material. Animals were placed on warming pad (37°C) and allowed to recover from surgery for 15-30 minutes prior to dosing.

**Experiment:** The arterial cannula was connected to a P23XL pressure transducer (Grass Technologies, West Warwick, RI) and the arterial pressure transducer and Unisensor urethral transducer catheter were connected to an Gould 13-6615-50 amplifier (Data Sciences International, St. Paul, MN) and Gould TA6000 recorder (Data Sciences International, St. Paul, MN) in parallel. All data was analyzed using Power Lab Chart version 5.0.2 (ADInstruments, Colorado Springs, CO) data acquisition system. Baseline IUP was allowed to stabilize and after which, single, slow bolus injections of Formula I (0.0032, 0.01, 0.032, 0.100, 0.316, 1.0 and 3.16 mg/kg, i.v., n=5-6;) or vehicle was administered followed by a 1.0 ml saline flush. Doses were given at 15 minute intervals or after double the time required for IUP to reach baseline measurements where IUP changes occurred. At the end of the experiment, the rabbits were euthanized by an overdose of pentobarbital sodium.

**Measurements:** The change in IUP and MAP from baseline were measured as well as the slope of the IUP response. MAP was first calculated according to the following formula, where P_{d} is diastolic pressure and Pₛ is systolic pressure: MAP = P_{d} + 1/3 (Pₛ - P_{d}). Pre-dose baseline values for IUP and MAP were assessed during a 2 min period just prior to vehicle or test compound administration. Post-dose values for IUP or MAP were determined during a 2 min period at the first peak in the IUP tracing following vehicle or test compound administration. The change in IUP and MAP induced by vehicle or test compound were then calculated by subtracting the pre-dose value from the post-dose value. The rate of decline (mmHg/min) in the IUP response was determined by taking the average slope during the 5 min. period immediately following the 2 min. efficacy measurement for the top three doses.

**Statistical Methods**: The primary objectives of this analysis are: (1) to compare each dose to the respective vehicle as to the changes from pre-dose in IUP and in MAP separately, (2) to estimate ED10mmHg and ED20 mmHg using the changes from pre-dose in IUP and in MAP separately, and 3) to calculate the Urethral Selectivity (MAP/IUP) at 10 mmHg and 20 mmHg. Statistical analysis of the rate of decline was not performed.

### Group Comparisons:

Analyses are performed for blood pressure and intraurethral pressure separately.

A repeated measure ANOVA including terms of Treatment (Vehicle and Drug), Time, Treatment by Time interaction, and variation within animal is performed with respect to the changes from pre-dose. Then, each dose is compared to the respective vehicle using a two-sample t test with equal or unequal variance assumption as to the changes from pre-dose.

### Curve Fitting Procedure:

Analyses are performed for blood pressure and intraurethral pressure separately.

Curve is fitted on the changes from pre-dose in IUP and in MAP.

A nonlinear mixed effect model with a compound symmetry variance-covariance structure is used.

A nonlinear mixed effect model with logistic dose response equation in the form of change from pre-dose=min+(maxmin)/(1+((max10)/(10min))*(ED10/dose)**Slope) is fit to the individual data points. ED10mmHg is the dose to achieve 10 mmHg in change from pre-dose.

The ED20mmHg is estimated by fitting the model change from pre-dose = min + (max-min)/(1+((max-20)/(20-min))*(ED20/dose)**Slope). ED20mmHg is the dose to achieve 20 mmHg in change from pre-dose.

### Urethral Selectivity (MAP/IUP)

Urethral Selectivity (MAP/IUP) at 10 mmHg = ED10 of MAP / ED 10 of IUP
Urethral Selectivity (MAP/IUP) at 20 mmHg = ED20 of MAP / ED20 of IUP

The compound of Formula I has been found to exhibit unexpectedly enhanced selectivity, for enhancement of intraurethral pressure (IUP) over blood pressure (MAP), as a partial agonist of alpha-1A adrenoceptors. The combination of the fluoro and methyl substituents on the 2- and 3-position of the phenyl ring, respectively, provide unexpected advantages over the general class of imidazolinylmethyl aryl sulfonamides in that it has both a favorable intrinsic activity, or efficacy, as a partial agonist, which is ideally between 0.35 to 0.60, of 0.46 and an affinity, or pEC50 value, of 6.2. As full agonist activity is undesirable due to hypertension related side effects, the combination of high affinity and partial agonist behavior is critical for optimization of urethral activity benefits associated with effective modulation of alpha-1A adrenoceptors coupled with minimization of diastolic blood pressure related side effects. Furthermore, the compound of Formula I, in comparison to analogue compounds, exhibits improved durability ofIUP response over time which is necessary for effective treatment of incontinence.

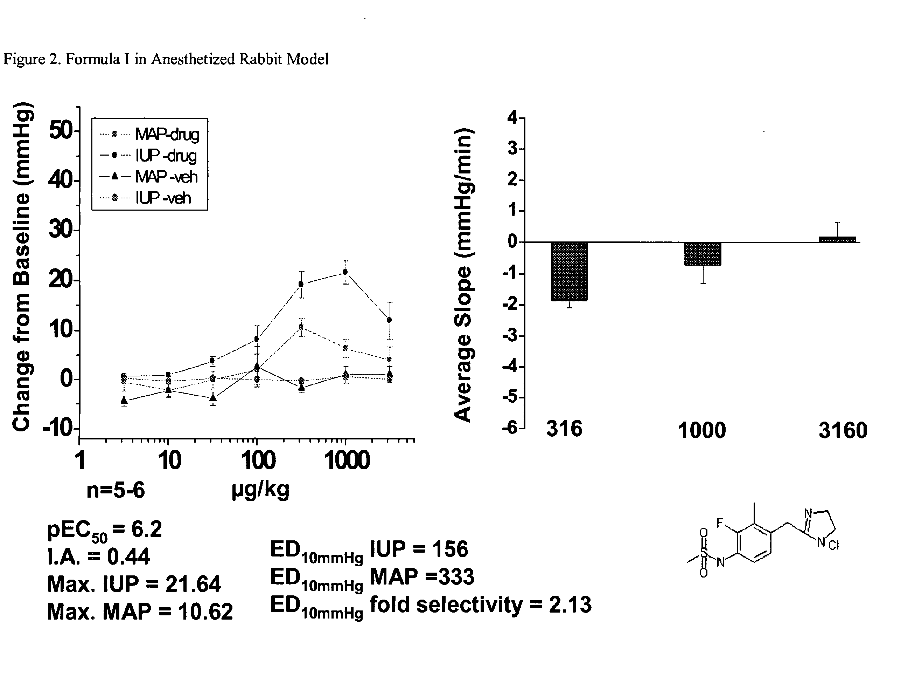

The compound of formula I tested in the anesthetized rabbit model exhibited not only 2.13 fold selectivity for enhancement of intraurethral pressure (IUP) over blood pressure (MAP) at the 10mmHg change in IUP level. The compound of formula I also has a correspondingly low maximum arterial blood pressure increase of only 10.62mmHg. Additionally, the compound of formula I also has an increased durability of IUP response over time (Figure 2). These characteristics, in combination, contribute to render the compound of formula I a remarkably superior pharmaceutical candidate over structurally similar analogues, both selectively, for enhancement of intraurethral pressure (IUP) over blood pressure (MAP), and effectively over time, as an alpha-1 A partial agonist for the treatment of incontinence.

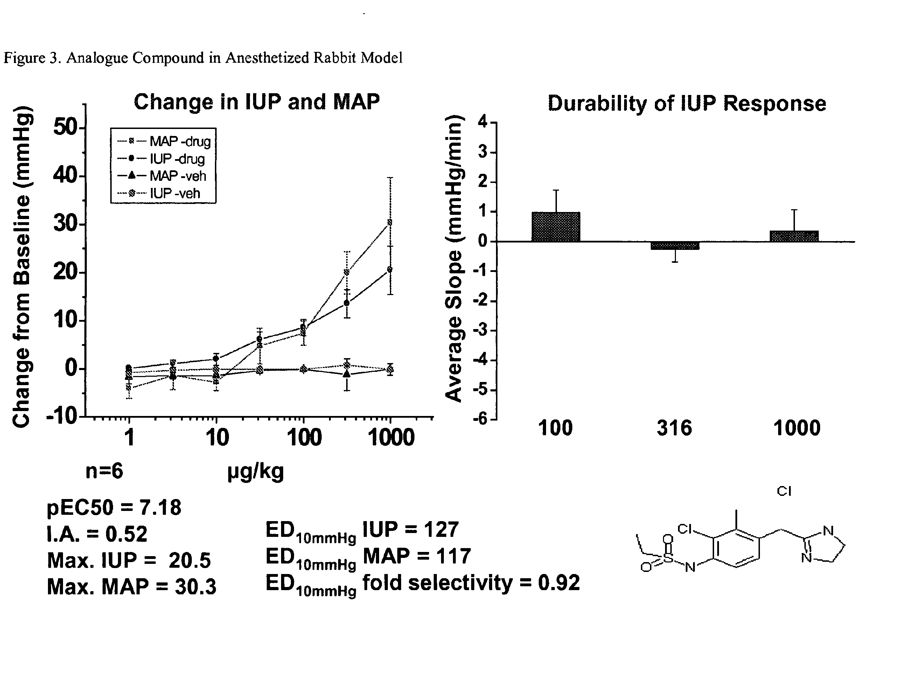

For example, in the same anesthetized rabbit model, an analogue, differing from the compound of formula I with the substitution of a 2-chloro for the 2-fluoro substituent on the phenyl ring and an ethyl instead of methyl sulfonamide group, displays no selectivity for enhancement of IUP over MAP of 0.92 at the 10mmHg change in IUP level. Further, the maximum increase in MAP for the analogue is much higher at 30.3mmHg (Figure 3).

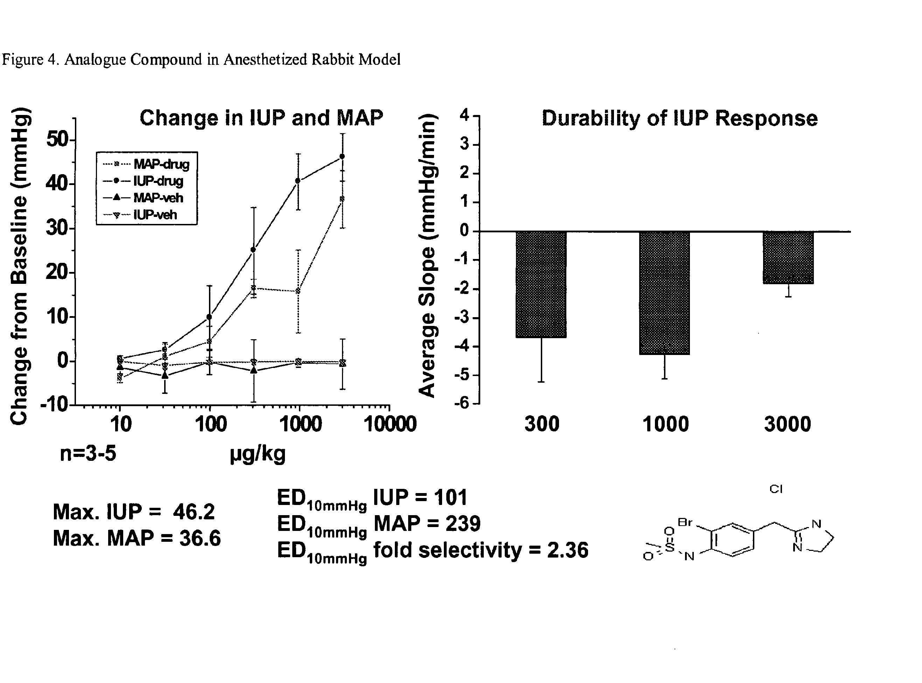

For example, in the same anesthetized rabbit model, an analogue, differing from the compound of formula I with the substitution of a 2-bromo for the 2-fluoro substituent and absence of the 3-methyl substitution on the phenyl ring, displays a comparable selectivity for enhancement of IUP over MAP of 2.36 at the 10nunHg change in IUP level. However, the maximum increase in MAP for the analogue is much higher at 36.6mmHg at the 10mmHg change in IUP level and the durability of IUP response over time is not sustained (Figure 4).

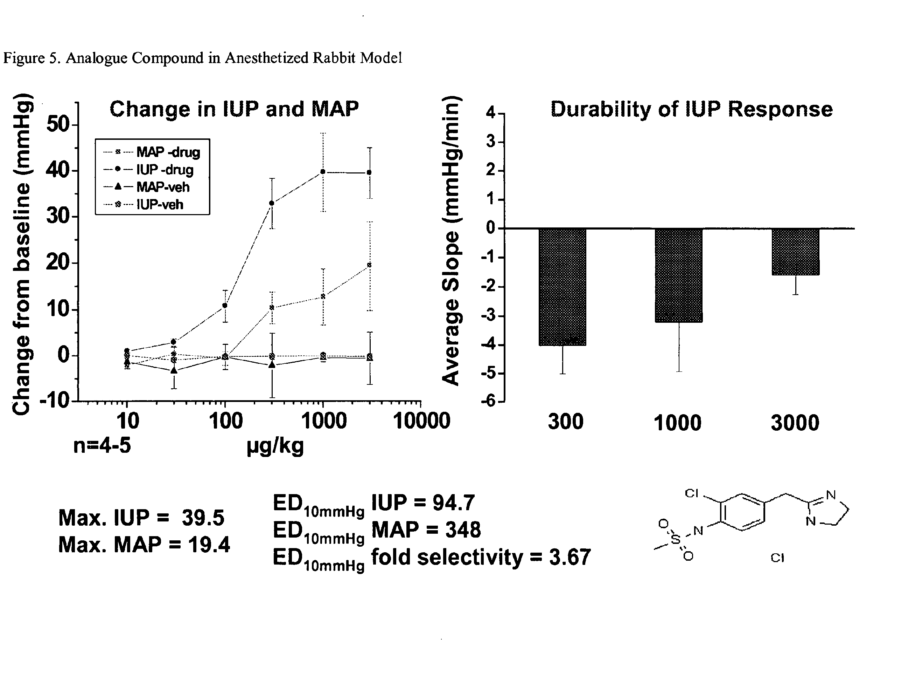

For example, in the same anesthetized rabbit model, an analogue, differing from the compound of formula I with the substitution of a 2-chloro for the 2-fluoro substituent and absence of the 3-methyl substitution on the phenyl ring. The maximum increase in MAP for the analogue is much higher at 19.4mmHg and the durability of IUP response is not sustained (Figure 5).

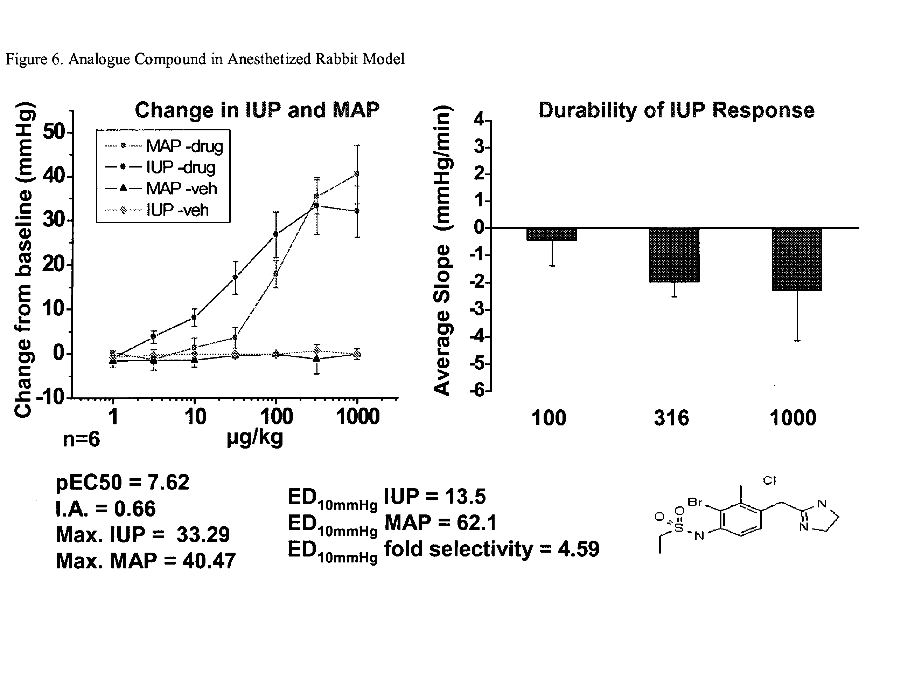

For example, in the same anesthetized rabbit model, an analogue differing from the compound of formula I with the substitution of a 2-bromo for the 2-flouro on the phenyl ring and an ethyl instead of methyl sulfonamide group. The maximum increase in MAP for the analogue is very high at 40.47mmHg and the durability ofIUP response over time is not sustained (Figure 6).

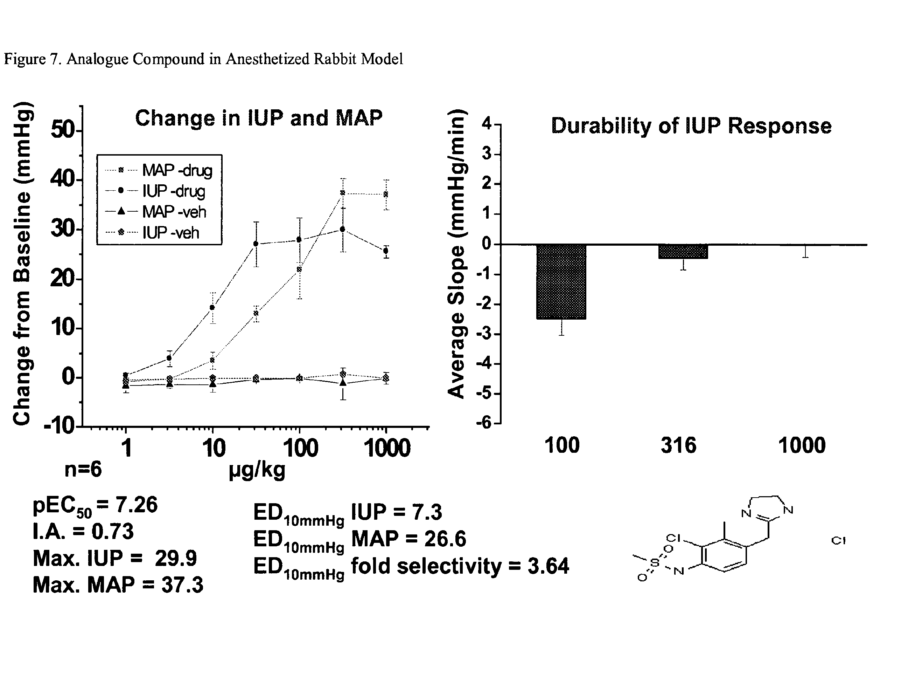

For example, in the same anesthetized rabbit model, an analogue, differing from the compound of formula I only with the substitution of a 2-chloro for the 2-fluoro substituent on the phenyl ring, the maximum increase in MAP for the analogue is much higher for the analogue at 37.3 mmHg (Figure 7).

The present invention includes pharmaceutical compositions comprising the compound of the present invention, or an individual isomer, racemic or non-racemic mixture of isomers or a pharmaceutically acceptable salt or solvate thereof, together with at least one pharmaceutically acceptable carrier, and optionally other therapeutic and/or prophylactic ingredients.

In general, the compound of the present invention will be administered in a therapeutically effective amount by any of the accepted modes of administration for agents that serve similar utilities. Suitable dosage ranges are typically 1-500 mg daily, preferably 1-100 mg daily, and most preferably 1-30 mg, depending upon numerous factors such as the severity of the disease to be treated, the age and relative health of the subject, the potency of the compound, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this Application, to ascertain a therapeutically effective amount of the compound of the present invention for a given disease.

In general, the compound of the present invention will be administered as pharmaceutical formulations including those suitable for oral (including buccal and sub-lingual), rectal, nasal, topical, pulmonary, vaginal, or parenteral (including intramuscular, intraarterial, intrathecal, subcutaneous and intravenous) administration or in a form suitable for administration by inhalation or insufflation. The preferred manner of administration is generally oral using a convenient daily dosage regimen which can be adjusted according to the degree of affliction.

The compound of the present invention, together with one or more conventional adjuvants, carriers, or diluents, may be placed into the form of pharmaceutical compositions and unit dosages. The pharmaceutical compositions and unit dosage forms may be comprised of conventional ingredients in conventional proportions, with or without additional active compounds or principles, and the unit dosage forms may contain any suitable effective amount of the active ingredient commensurate with the intended daily dosage range to be employed. The pharmaceutical compositions may be employed as solids, such as tablets or filled capsules, semisolids, powders, sustained release formulations, or liquids such as solutions, suspensions, emulsions, elixirs, or filled capsules for oral use; or in the form of suppositories for rectal or vaginal administration; or in the form of sterile injectable solutions for parenteral use. Formulations containing about one (1) milligram of active ingredient or, more broadly, about 0.01 to about one hundred (100) milligrams, per tablet, are accordingly suitable representative unit dosage forms.

The compound of the present invention may be formulated in a wide variety of oral administration dosage forms. The pharmaceutical compositions and dosage forms may comprise the compound the present invention or pharmaceutically acceptable salts thereof as the active component. The pharmaceutically acceptable carriers may be either solid or liquid. Solid form preparations include powders, tablets, pills, capsules, cachets, suppositories, and dispersible granules. A solid carrier may be one or more substances which may also act as diluents, flavoring agents, solubilizers, lubricants, suspending agents, binders, preservatives, tablet disintegrating agents, or an encapsulating material. In powders, the carrier generally is a finely divided solid which is a mixture with the fmely divided active component. In tablets, the active component generally is mixed with the carrier having the necessary binding capacity in suitable proportions and compacted in the shape and size desired. The powders and tablets preferably contain from about one (1) to about seventy (70) percent of the active compound. Suitable carriers include but are not limited to magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier, providing a capsule in which the active component, with or without carriers, is surrounded by a carrier, which is in association with it. Similarly, cachets and lozenges are included. Tablets, powders, capsules, pills, cachets, and lozenges may be as solid forms suitable for oral administration.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, e.g., in aqueous propylene glycol solutions or may contain emulsifying agents, e.g., such as lecithin, sorbitan monooleate, or acacia. Aqueous solutions can be prepared by dissolving the active component in water and adding suitable colorants, flavors, stabilizing, and thickening agents. Aqueous suspensions can be prepared by dispersing the finely divided active component in water with viscous material, such as natural or synthetic gums, resins, methylcellulose, sodium carboxymethylcellulose, and other well known suspending agents. Solid form preparations include solutions, suspensions, and emulsions, and may contain, in addition to the active component, colorants, flavors, stabilizers, buffers, artificial and natural sweeteners, dispersants, thickeners, solubilizing agents, and the like.

The compound of the present invention may be formulated for parenteral administration (e.g., by injection, e.g. bolus injection or continuous infusion) and may be presented in unit dose form in ampoules, pre-filled syringes, small volume infusion or in multi-dose containers with an added preservative. The composition may take such form as a suspension, solution, or emulsion in oily or aqueous vehicles, e.g. a solution in aqueous polyethylene glycol. Examples of oily or nonaqueous carriers, diluents, solvents or vehicles include propylene glycol, polyethylene glycol, vegetable oils (e.g., olive oil), and injectable organic esters (e.g., ethyl oleate), and may contain formulatory agents such as preserving, wetting, emulsifying or suspending, stabilizing and/or dispersing agents. Alternatively, the active ingredient may be in powder form, obtained by aseptic isolation of sterile solid or by lyophilisation from solution for constitution before use with a suitable vehicle, e.g., sterile, pyrogen-free water.

The compound of the present invention may be formulated for topical administration to the epidermis as ointments, creams or lotions, or as a transdermal patch. Ointments and creams may, e.g., be formulated with an aqueous or oily base with the addition of suitable thickening and/or gelling agents. Lotions may be formulated with an aqueous or oily base and will in general also containing one or more emulsifying agents, stabilizing agents, dispersing agents, suspending agents, thickening agents, or coloring agents. Formulations suitable for topical administration in the mouth include lozenges comprising active agents in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier.

The compound of the present invention may be formulated for administration as suppositories. A low melting wax, such as a mixture of fatty acid glycerides or cocoa butter is first melted and the active component is dispersed homogeneously, e.g., by stirring. The molten homogeneous mixture is then poured into convenient sized molds, allowed to cool, and to solidify.

The compound of the present invention may be formulated for vaginal administration. Pessaries, tampons, creams, gels, pastes, foams or sprays containing in addition to the active ingredient such carriers as are known in the art to be appropriate.

The compound of the present invention may be formulated for nasal administration. The solutions or suspensions are applied directly to the nasal cavity by conventional means, e.g., with a dropper, pipette or spray. The formulations may be provided in a single or multidose form. In the latter case of a dropper or pipette, this may be achieved by the patient administering an appropriate, predetermined volume of the solution or suspension. In the case of a spray, this may be achieved e.g. by means of a metering atomizing spray pump.

The compound of the present invention may be formulated for aerosol administration, particularly to the respiratory tract and including intranasal administration. The compound will generally have a small particle size e.g. of the order of five (5) microns or less. Such a particle size may be obtained by means known in the art, e.g. by micronization. The active ingredient is provided in a pressurized pack with a suitable propellant such as a chlorofluorocarbon (CFC), e.g., dichlorodifluoromethane, trichlorofluoromethane, or dichlorotetrafluoroethane, or carbon dioxide or other suitable gas. The aerosol may conveniently also contain a surfactant such as lecithin. The dose of drug may be controlled by a metered valve. Alternatively the active ingredients may be provided in a form of a dry powder, e.g. a powder mix of the compound in a suitable powder base such as lactose, starch, starch derivatives such as hydroxypropylmethyl cellulose and polyvinyl-pyrrolidine (PVP). The powder carrier will form a gel in the nasal cavity. The powder composition may be presented in unit dose form e.g. in capsules or cartridges of e.g., gelatin or blister packs from which the powder may be administered by means of an inhaler.

When desired, formulations can be prepared with enteric coatings adapted for sustained or controlled release administration of the active ingredient. For example, the compound of the present invention can be formulated in transdermal or subcutaneous drug delivery devices. These delivery systems are advantageous when sustained release of the compound is necessary and when patient compliance with a treatment regimen is crucial. Compounds in transdermal delivery systems are frequently attached to an skin-adhesive solid support. The compound of interest can also be combined with a penetration enhancer, e.g., Azone (1-dodecylaza-cyclo-heptan-2-one). Sustained release delivery systems are inserted subcutaneously into to the subdermal layer by surgery or injection. The subdermal implants encapsulate the compound in a lipid soluble membrane, e.g., silicone rubber, or a biodegradable polymer, e.g., polyactic acid.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

Other suitable pharmaceutical carriers and their formulations are described in Remington: The Science and Practice of Pharmacy 1995, edited by Martin, Mack Publishing Company, 19th edition, Easton, Pennsylvania. Representative pharmaceutical formulations containing the compound of the present invention are described in the Examples.

The following preparations and examples are given to enable those skilled in the art to more clearly understand and to practice the present invention. They should not be considered as limiting the scope of the invention, but merely as being illustrative and representative thereof.

Efforts have been made to ensure accuracy with respect to numbers used (e.g., amounts, temperatures, etc.), but some experimental error and deviation should, of course, be allowed for as well as due to differences such as, e.g., in calibration, rounding of numbers, and the like.

## Claims

1. A compound of the formula I: 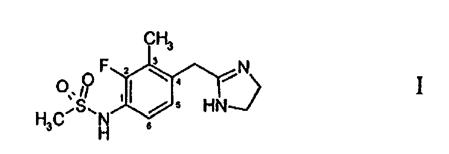 or a pharmaceutically acceptable salt thereof.

2. The compound of claim 1, wherein the pharmaceutically acceptable salt is hydrochloride.

3. A composition comprising the compound of claim 1 and further comprising a pharmaceutically acceptable carrier.

4. The use of the compound of claim 1 in the preparation of a medicament for preventing, alleviating, or treating a disorder selected from urge incontinence, stress incontinence, overflow incontinence, and functional incontinence.

## Patentansprüche

1. Eine Verbindung der Formel I: 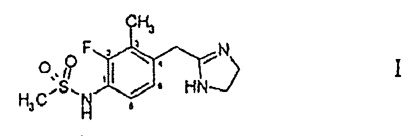 oder ein pharmazeutisch verträgliches Salz davon.

2. Die Verbindung von Anspruch 1, wobei das pharmazeutisch verträgliche Salz Hydrochlorid ist.

3. Eine Zusammensetzung, umfassend die Verbindung von Anspruch 1 und weiter umfassend einen pharmazeutisch verträglichen Träger.

4. Die Verwendung der Verbindung von Anspruch 1 bei der Herstellung eines Medikaments zur Prävention, Linderung oder Behandlung einer Störung, ausgewählt aus Dranginkontinenz, Stressinkontinenz, paradoxer Harninkontinenz und funktioneller Inkontinenz.

## Revendications

1. Composé de formule I . 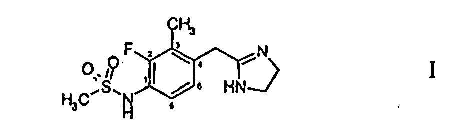 ou un de ses sels pharmaceutiquement acceptables.

2. Composé suivant la revendication 1, dans lequel le sel pharmaceutiquement acceptable est le chlorhydrate.

3. Composition comprenant le composé de la revendication 1 et comprenant en outre un support pharmaceutiquement acceptable.

4. Utilisation du composé de la revendication 1 dans la préparation d'un médicament pour la prévention, le soulagement ou le traitement d'un trouble choisi entre l'incontinence impulsive, l'incontinence due au stress, l'incontinence par regorgement et l'incontinence fonctionnelle.
